# EUROPEAN PATENT APPLICATION

(11) **EP 0 801 048 A1**
(43) Date of publication of application: **15.10.1997**
(21) Application number: 96105553.0
(22) Date of filing: 09.04.1996
(51) Int. Cl.: C07C 29/40, C07C 33/18, C07C 33/24, C07C 33/46, C07C 41/30, C07C 43/253, C07C 43/295, C07C 209/68, C07C 215/68

(54) **Process for producing benzhydrols**

(71) Applicant: K I CHEMICAL INDUSTRY CO., LTD., Iwata-gun, Shizuoka-ken (JP)
(72) Inventor: Tumaki, Hidetoshi, c/o K.I Chem. Ind. Co., Ltd., Iwata-gun, Shizuoka-ken (JP)
(74) Representative: Boeters, Hans Dietrich, Dr.

(57) **Abstract**

There is disclosed a process for producing benzhydrls by reacting an arylmetal compound with an aromatic aldehyde. According to this process, benzhydrols useful as raw materials for medicines and photopolymerization initiators can be produced in a high yield and industrially advantageously.

## Description

### FIELD OF THE INVENTION

The present invention relates to a novel process for producing benzhydrols useful as raw materials for medicines and photopolymerization initiators.

### BACKGROUND OF THE INVENTION

Benzhydrols are useful substances as raw materials for medicines (e.g. antihistamines, vasodilators, and antiallergic agents) and photopolymerization initiators. In conventional processes for industrially producing them, benzophenones are reduced in various manners. Although as a reduction process, for example, one that uses hydrogenation or metal hydrides is generally carried out, in many cases the reaction conditions are severe or economical problems are involved. Also, in many cases benzophenones, i.e. the raw materials, are themselves not necessarily easily available. That is, benzophenones are generally synthesized by the Friedel-Crafts reaction, and since the regioselectivity and the selectivity for functional groups of the reaction are poor, to obtain the intended product requires a great deal of effort and the types of substituents are restricted. In addition to the above, aluminum chloride, used as a catalyst in this reaction, sometimes leads to problems at discarding it after use. On the other hand, as a simple process for synthesizing benzhydrols, a process that uses an organic tellurium compound is also reported (e.g., L. -L. Shi et al., J. Chem. Soc. Perkin. Trans. 1, 2847 (1990)). In this unique process, an organic lithium compound is made to interact with an aryltellurium compound, and the aryl lithium compound produced by the lithium-tellurium exchange reaction is reacted with an aromatic aldehyde, to obtain benzhydols. However, it cannot be said that this process is industrially advantageous, because of the toxicity of the organic tellurium compound, and from an economical point of view.

### SUMMARY OF THE INVENTION

The present invention has been made to solve the above-described conventional problems in producing benzhydrols, and an object of the present invention is to provide a novel process for producing benzhydrols in a high yield and industrially advantageously.

Other and further objects, features, and advantages of the invention will appear more evident form the following description.

### DETAILED DESCRIPTION OF THE INVENTION

Under the above circumstances, the present inventor has studied the reaction of arylmetal compounds with aromatic aldehydes, which has been ignored conventionally despite that the process seems to be industrially advantageous, and I have found that the reaction can produce benzhydrols in a high yield, completing the present invention.

That is, the present invention provides a process for producing benzhydrols represented by formula (III): 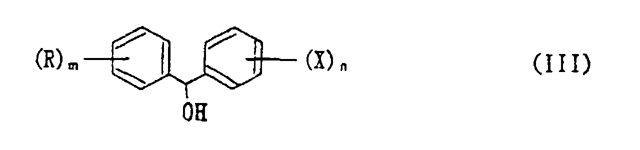 wherein R's, which are the same or different, each represent a hydrogen atom or an alkyl group; m is an integer of 1 to 5; X's, which are the same or different, each represent a hydrogen atom, or a halogen atom, a lower alkyl group, a lower alkoxy group, a lower dialkylamino group, a halogenated lower alkyl group or an aryloxy group (hereinafter referred to as a substituent group A); two adjacent X's may bond together to form a condensed benzene ring (in this case, in this specification, those are included when the thus formed naphthalene ring has as a substituent a group selected from the above substituent group A); and n is an integer of 1 to 5, comprising reacting arylmetal compounds represented by formula (I): 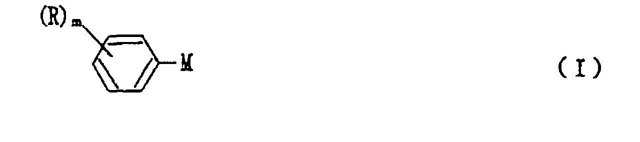 wherein R's have the same meaning as defined above, M represents an alkali metal, and m has the same meaning as defined above, with aromatic aldehydes represented by formula (II): 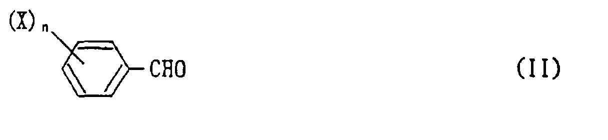 wherein X's and n have the same meanings as defined above.

When, in formula (I), R represents an alkyl group, preferably the alkyl group is one having carbon atoms of 1 to 8 (more preferably 1 to 6), for example, a methyl, ethyl, propyl, butyl, pentyl, or hexyl group, and more preferably it is a C₁-C₄ alkyl group. m is an integer of 1 to 5, preferably 1 to 3. M represents an alkali metal, preferably sodium or potassium, and more preferably sodium.

The arylmetal compounds represented by formula (I) can be prepared by reacting the corresponding aryl halides with alkali metals; for example, by reacting a aryl halide with a dispersion of an alkali metal in a solvent, such as toluene.

In formula (II), preferable substituents represented by X, besides a hydrogen atom, that can be mentioned are a halogen atom (e.g. fluorine, chlorine, bromine, and iodine), a lower alkyl group (an alkyl group preferably having carbon atoms of 1 to 6, e.g. methyl, ethyl, propyl, isopropyl, butyl, isobutyl, tert-butyl, pentyl, and hexyl), a lower alkoxy group (an alkoxy group preferably having carbon atoms of 1 to 6, e.g. methoxy, ethoxy, propoxy, isopropoxy, and butoxy), a lower dialkylamino group (its alkyl group preferably having carbon atoms of 1 to 3, e.g. dimethylamino, diethylamino, methylethylamino, and diisopropylamino), a halogenated lower alkyl group (a halogenated alkyl group preferably having carbon atoms of 1 or 2, e.g. trifluoromethyl and pentafluoroethyl), and an aryloxy group (e.g. phenoxy). Further, two adjacent X's may bond together to form a condensed benzene ring, and specific examples of the compound thus represented by formula (II) are 1-naphthaldehyde and 2-naphthaldehyde. n is an integer of 1 to 5, preferably 1 to 3.

The amount of the arylmetal compound represented by formula (I) to be used in the production process of the present invention is generally in the range of 1.0 to 1.5 mol, and preferably in the range of 1.05 to 1.20 mol, for 1 mol of the aromatic aldehyde represented by formula (II). In the reaction of the present invention, suitably a solvent can be used. Examples of the solvent are aromatic hydrocarbons, such as benzene, toluene, xylene, and ethylbenzene; and ethers, such as tetrahydrofuran, 1,4-dioxane, and dimethoxyethane; and inter alia toluene is preferable. The amount of the solvent to be used can be set to an arbitrary amount, with preference given to a range of 2 to 20 times the amount of the compound of formula (I) by weight. The compound of formula (II) can be diluted with and dissolved in a particular solvent as required, and in that case desirably the solvent is used approximately in the same amount as that of the compound of formula (II) by weight. In the present invention, the reaction can be carried out generally at a temperature between -50 °C to 30 °C, and particularly preferably at a temperature between -10 °C and 10 °C. After the reaction, a usual process, such as distillation and crystallization, can be carried out to obtain the intended product (III).

According to the present invention, benzhydrols useful as raw materials for medicines and photopolymerization initiators can be obtained inexpensively and in a high yield. Therefore the present process is an industrially quite excellent production process of benzhydrols.

### EXAMPLES

Now the present invention will be described more specifically with reference to examples, but the present invention is not restricted to these.

### Example 1 (Preparation of benzhydrol)

To phenylsodium, prepared from 40.7 g (1.770 mol) of a sodium dispersion and 97.2 g (0.807 mol) of chlorobenzene in 800 g of toluene, was added, dropwise, a solution of 82.9 g (0.781 mol) of benzaldehyde in toluene (80 g), over 1 hour with the temperature kept at -10 °C to -5 °C. After stirring at that temperature for another 1 hour, hydrolysis with 600 ml of 20%-diluted hydrochloric acid was carried out. The brilliant yellow organic layer was separated and washed with water three times. After the organic layer was concentrated, the obtained white needle crystals were recrystallized from toluene, to give 107.1 g (0.581 mol, 74.5%) of the intended product (m.p.: 65.5 to 67.0 °C).

### Example 2 (Preparation of 3-chlorobenzhydrol)

To phenylsodium, prepared from 9.2 g (0.400 mol) of a sodium dispersion and 22.0 g (0.196 mol) of chlorobenzene in 200 g of toluene, was added, dropwise, a solution of 24.8 g (0.176 mol) of 3-chlorobenzaldehyde in toluene (24 g), with the temperature kept at 0 °C to 3 °C. After stirring at that temperature for 1 hour, hydrolysis with 100 ml of 20%-diluted hydrochloric acid was carried out, and the obtained organic layer was separated and washed with water three times. After the solvent was concentrated, the remained pale orange oil was distilled under reduced pressure, to give 34.9 g (0.160 mol, 91.0%) of the intended product (b.p.: 147.0 to 148.0 °C/2 mmHg).

### Example 3 (Preparation of 4-chlorobenzhydrol)

To phenylsodium, prepared from 38.6 g (1.678 mol) of a sodium dispersion and 92.3 g (0.823 mol) of chlorobenzene in 575 g of toluene, was added, dropwise, a solution of 104.1 g (0.741 mol) of 4-chlorobenzaldehyde in toluene (100 g), with the temperature kept at -10 °C to 0 °C. After stirring at that temperature for another 1 hour, hydrolysis with 400 ml of 20%-diluted hydrochloric acid was carried out. After the organic layer was washed with water five times, the solvent was concentrated, and the obtained yellow oil was distilled under reduced pressure, to give 126.7 g (0.581 mol, 78.4%) of the intended product (b.p.: 145.0 to 149.0 °C/3 mmHg).

### Example 4 (Preparation of 2,6-dichlorobenzhydrol)

To phenylsodium, prepared from 2.7 g (0.117 mol) of a sodium dispersion and 6.4 g (0.057 mol) of chlorobenzene in 45 g of toluene, was added, dropwise, a solution of 8.2 g (0.057 mol) of 2,6-dichlorobenzaldehyde in toluene (10 g), with the temperature kept at 0 °C to 5 °C. After stirring at that temperature for 1 hour, the obtained black slurry was hydrolyzed with 30 ml of 20%-diluted hydrochloric acid. After the organic layer was separated and washed with water three times, the solvent was distilled off. The residual viscous oil was distilled by Kugelrohr under reduced pressure, to give 11.9 g (0.047 mol, 90.8%) of the intended product (b.p.: 200 °C (oven temp.)/1 mmHg).

### Example 5 (Preparation of 4-trifluoromethylbenzhydrol)

To phenylsodium, prepared from 2.6 g (0.111 mol) of a sodium dispersion and 6.1 g (0.054 mol) of chlorobenzene in 43 g of toluene, was added, dropwise, a solution of 8.6 g (0.049 mol) of 4-trifluoromethylbenzaldehyde in toluene (10 g), with the temperature kept at 0 °C to 4 °C. After the dark green slurry was stirred at that temperature for 1 hour, it was hydrolyzed with 30 ml of 20%-diluted hydrochloric acid, and then the organic layer was washed with water three times. After the solvent was removed, the precipitated white crystals were washed with n-hexane, to give 10.5 g (0.043 mol, 88.3%) of the intended product (m.p.: 63.5 to 65.0 °C)

### Example 6 (Preparation of 2,5-dimethoxybenzhydrol)

To phenylsodium, prepared from 3.6 g (0.155 mol) of a sodium dispersion and 8.5 g (0.076 mol) of chlorobenzene in 60 g of toluene, was added, dropwise, a solution of 11.4 g (0.069 mol) of 2,5-dimethoxybenzaldehyde in toluene (25 g), with the temperature kept at 0 °C to 5 °C. After stirring the grayish white slurry at that temperature for 1 hour, it was hydrolyzed with 100 ml of 20%-diluted hydrochloric acid, and the organic layer was washed with water three times. The solvent was distilled off. The residual yellow oil was distilled by Kügelrohr under reduced pressure, to give 15.5 g (0.063 mol, 92.5%) of the intended product (b.p.: 180 °C (oven temp.)/1 mmHg).

### Example 7 (Preparation of 4-dimethylaminobenzhydrol)

To phenylsodium, prepared from 3.1 g (0.134 mol) of a sodium dispersion and 7.4 g (0.066 mol) of chlorobenzene in 52 g of toluene, was added, dropwise, a solution of 8.8 g (0.059 mol) of 4-dimethylaminobenzaldehyde in toluene (20 g), with the temperature kept at 0 °C to 5 °C. After the blackish brown slurry was stirred at that temperature for 1 hour, it was hydrolyzed with 100 ml of water. After the organic layer was washed with water three times, the solvent was distilled off, to give orange crystals, and the crystals were washed with cold toluene, to give 11.5 g (0.051 mol, 85.7%) of the intended white product (m.p.: 67.5 to 69.0 °C).

### Example 8 (Preparation of 4-methoxy-4'-methylbenzhydrol)

To 4-methylphenylsodium, prepared from 8.5 g (0.370 mol) of a sodium dispersion and 22.9 g (0.181 mol) of p-chlorotoluene in 200 g of toluene, was added, dropwise, a solution of 22.8 g (0.167 mol) of anisaldehyde in toluene (23 g), with the temperature kept at 0 °C to 5 °C. The gray slurry was stirred at that temperature for 1 hour; then it was hydrolyzed with 150 ml of 20%-diluted hydrochloric acid, and the organic layer was washed with water three times. After the solvent was distilled off, the deposited white granular crystals were washed with cold toluene, to give 29.4 g (0.129 mol, 77.3%) of the intended product (m.p.: 63.0 to 64.0 °C).

### Example 9 (Preparation of 2,4'-dimethylbenzhydrol)

To 4-methylphenylsodium, prepared from 4.7 g (0.205 mol) of a sodium dispersion and 12.6 g (0.100 mol) of p-chlorotoluene in 70 g of toluene, was added, dropwise, a solution of 10.8 g (0.090 mol) of 2-methylbenzaldehyde in toluene (15 g), with the temperature kept at 0 °C to 4 °C. The blackish gray slurry was stirred at that temperature for 1 hour, and it was hydrolyzed with 100 ml of 20%-diluted hydrochloric acid. After the organic layer was washed with water three times, the solvent was distilled off, to obtain an orange oil, and the oil was distilled under reduced pressure by Kügelrohr, to give 17.0 g (0.080 mol, 86.4%) of the intended product (b.p.: 210 °C (oven temp.)/2 mmHg).

### Example 10 (Preparation of 4-methyl-3'-phenoxybenzhydrol)

To 4-methylphenylsodium, prepared from 3.5 g (0.154 mol) of a sodium dispersion and 9.5 g (0.075 mol) of p-chlorotoluene in 50 g of toluene, was added, dropwise, a solution of 12.1 g (0.061 mol) of 3-phenoxybenzaldehyde in toluene (10 g), with the temperature kept at 0 °C to 5 °C. After stirring at that temperature for 1 hour, it was hydrolyzed with 50 ml of 20%-diluted hydrochloric acid. After the organic layer was washed with water three times, the solvent was distilled off, and the residual oil was distilled by Kügelrohr under reduced pressure, to give 14.8 g (0.051 mol, 83.6%) of the intended product (b.p.: 310 °C (oven temp.)/2 mmHg).

### Example 11 (Preparation of 3-bromo-4'-methylbenzhydrol)

To 4-methylphenylsodium, prepared from 4.8 g (0.210 mol) of a sodium dispersion and 13.3 g (0.105 mol) of p-chlorotoluene in 50 g of toluene, was added, dropwise, a solution of 18.3 g (0.099 mol) of 3-bromobenzaldehyde in toluene (15 g), with the temperature kept at 0 °C to 5 °C. The blackish gray slurry was stirred at that temperature for 1 hour; then it was hydrolyzed with 150 ml of 20%-diluted hydrochloric acid, and the organic layer was washed with water three times. After the solvent was distilled off, the precipitated white needle crystals were washed with n-hexane, to give 25.3 g (0.091 mol, 92.3%) of the intended product (m.p.: 42.0 to 44.0 °C).

### Example 12 (Preparation of 2,4,6-trimethylbenzhydrol)

To 2,4,6-trimethylphenylsodium, prepared from 7.8 g (0.339 mol) of a sodium dispersion and 33.1 g (0.166 mol) of bromomesitylene in 170 g of toluene, was added, dropwise, a solution of 19.6 g (0.184 mol) of benzaldehyde in toluene (19 g), with the temperature kept at -3 °C to 2 °C. After stirring at that temperature for 1 hour, hydrolysis with 100 ml of 20%-diluted hydrochloric acid was carried out, and the organic layer was washed with water three times. After the solvent was distilled off, the residual yellow oil was distilled under reduced pressure, to give 15.8 g (0.070 mol, 42.2%) of the intended product (b.p.: 161 to 163 °C/2 mmHg).

### Example 13 (Preparation of α-naphthylbenzhydrol)

To phenylsodium, prepared from 9.2 g (0.400 mol) of a sodium dispersion and 22.0 g (0.196 mol) of chlorobenzene in 200 g of toluene, was added, dropwise, a solution of 27.5 g (0.176 mol) of 1-naphthaldehyde in toluene (27 g), with the temperature kept at 0 °C to 3 °C. After stirring at that temperature for 1 hour, hydrolysis with 100 ml of 20%-diluted hydrochloric acid was carried out, and the organic layer was separated and washed with water three times. After the solvent was concentrated, the obtained pale yellowish white crystals were recrystallized from cyclohexane, to give 32.2 g (0.137 mol, 78.0%) of the intended product (m.p.: 87.5 to 88 °C).

Having described our invention as related to the present embodiments, it is our intention that the invention not be limited by any of the details of the description, unless otherwise specified, but rather be construed broadly within its spirit and scope as set out in the accompanying claims.

## Claims

1. A process for producing benzhydrols represented by formula (III): 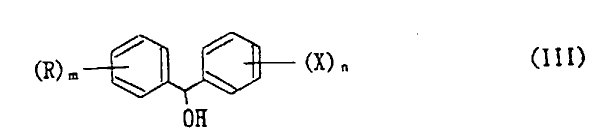 wherein R's, which are the same or different, each represent a hydrogen atom or an alkyl group; m is an integer of 1 to 5; X's, which are the same or different, each represent a hydrogen atom, or a halogen atom, a lower alkyl group, a lower alkoxy group, a lower dialkylamino group, a halogenated lower alkyl group or an aryloxy group ; two adjacent X's may bond together to form a condensed benzene ring; and n is an integer of 1 to 5, comprising reacting an arylmetal compound represented by formula (I): 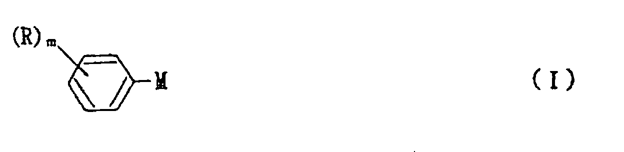 wherein R's have the same meaning as defined above, M represents an alkali metal, and m has the same meaning as defined above, with an aromatic aldehyde represented by formula (II): 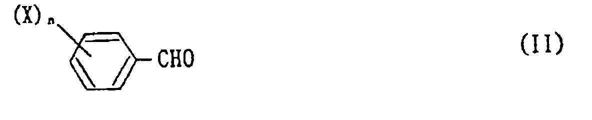 wherein X's and n have the same meanings as defined above.

2. The process for producing benzhydrols as claimed in claim 1, wherein the amount of the arylmetal compound of formula (I) to be used is in the range of 1.0 to 1.5 mol, for 1 mol of the aromatic aldehyde of formula (II).

3. The process for producing benzhydrols as claimed in claim 1, wherein the reaction is carried out in a solvent.

4. The process for producing benzhydrols as claimed in claim 3, wherein the solvent is an aromatic hydrocarbon or an ether.

5. The process for producing benzhydrols as claimed in claim 3, wherein the amount of the solvent to be used is in the range of 2 to 20 times the amount of the arymetal compound of formula (I) by weight.

6. The process for producing benzhydrols as claimed in claim 3, wherein the aromatic aldehyde of formula (II) is diluted with and dissolved in the solvent, the solvent being approximately in the same amount as that of the aromatic aldehyde of formula (II) by weight.

7. The process for producing benzhydrols as claimed in claim 1, wherein the reaction is carried out in a temperature in the range of -50 °C to 30 °C.
